# EUROPEAN PATENT APPLICATION

(11) **EP 2 384 734 A2**
(43) Date of publication of application: **09.11.2011**
(21) Application number: 10252172.1
(22) Date of filing: 20.12.2010
(51) Int. Cl.: A61K 8/02, A61Q 19/00

(54) **Transparent facial treatment mask**

(30) Priority: 21.12.2009 US 643245
(71) Applicant: Johnson & Johnson Consumer Companies, Inc., Skillman, NJ 08558 (US)
(72) Inventor: Aledo, Eduardo C. A., Princeton, NJ 08540 (US); Beatty, Heidi, Old Tappan, NJ 07675 (US)
(74) Representative: Kirsch, Susan Edith

(57) **Abstract**

The present invention features a facial mask comprising a water-insoluble, nonwoven substrate sized and shaped to lie against the face of a human user having an Average Wet Transparency Percentage of at least about 25% and/or a Percent Increase in Transparency of at least about 50%. Methods of treating the skin using facial masks are also provided.

## Description

### FIELD OF THE INVENTION

The present invention relates to a facial mask, and more particularly, to a facial mask having a level of transparency when exposed to a wetting agent.

### BACKGROUND OF THE INVENTION

Products such as cleansers and moisturizers formulated with vitamins and other skin benefit agents have been used for many years to treat the skin. Employing a water-insoluble substrate such as wipe or mask to assist in the process of cleansing, moisturizing and delivery of certain benefit agents to the skin is also known. For example, consumers typically use hydrating facial mask products for treatment of various skin conditions as well as to improve the physical appearance and texture of the facial skin. This can be accomplished while the user relaxes, such as in a prone position, while the mask contacts the skin of the face, and provides benefits thereto.

A conventional facial mask spread out fully on a face will completely block the user's view of the skin. This is problematic especially for users who want to see their skin through the mask to ensure that the mask has made complete contact with the skin. Non-transparent masks do not allow a user to know whether there are voids or bubbles between the mask and the skin. This could give rise to uneven treatment of the face by the skin care composition contained in the mask. Such uneven treatment is especially problematic for facial masks designed to lighten or even the tone of the skin. Furthermore, conventional opaque facial masks provide undesirable aesthetics to a user who would like to be seen through the mask.

It is known in the art to provide some transparency in substrates. For instance, EP 0357185, US2004/020953 and US6998017 disclose such characteristics. US 6998017 relates to a method of making a three-dimensional tissue having transparent and opaque regions on a mask. EP 0357185 relates to a foam coated protective apparel fabric having a semi-transparent substrate.

Applicants have now developed a facial mask that is transparent. The facial mask comprises a water-insoluble, nonwoven substrate sized and shaped to lie against the face of a human user, wherein said substrate has an Average Wet Transparency Percentage of at least about 25% and/or a Percent Increase in Transparency of at least about 50%.

Applicants have surprisingly found that by adjusting the composition and structure of the material from which a facial mask is made (e.g., pulp content, basis weight, etc.) a level of transparency can be produced when a wetting agent (e.g. water, micro-emulsion) is applied to the material.

### BRIEF DESCRIPTION OF THE DRAWINGS

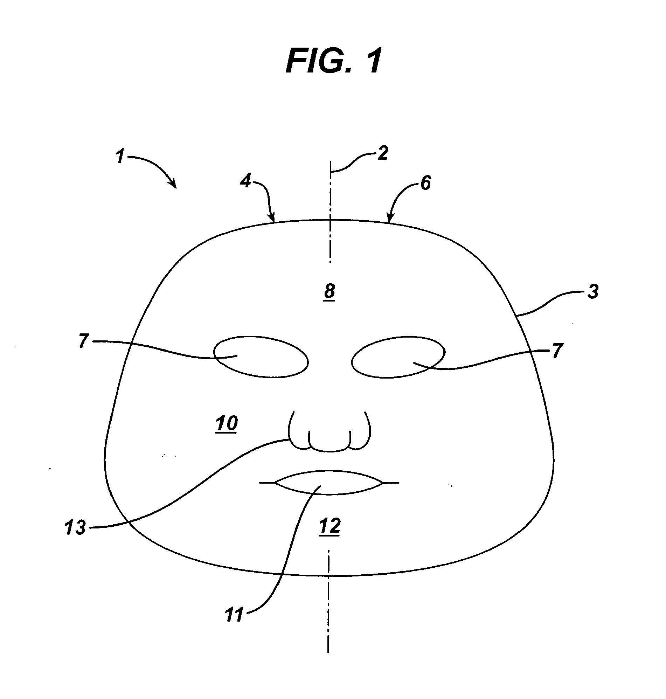

**FIG. 1** is a top plan view of a facial mask in accordance with the present invention.

### SUMMARY OF THE INVENTION

In one aspect of the invention, a facial mask for treating skin comprising a water-insoluble, nonwoven substrate sized and shaped to lie against a face of a human user, wherein at least a portion of said facial mask has an Average Wet Transparency Percentage of at least about 25%, and/or a Percent Increase in Transparency of at least about 50%, is provided.

In a second aspect of the invention, a cosmetic method of treating facial skin by applying the above mask is provided.

Other features and advantages of the present invention will be apparent from the detailed description of the invention and from the claims.

### DETAILED DESCRIPTION OF THE INVENTION

According to the present invention, a transparent facial mask is provided. In particular, the facial mask of the present invention comprises a water-insoluble, nonwoven substrate. In one embodiment of the invention, the facial mask has an Average Wet Transparency Percentage of at least about 25% when exposed to a wetting agent. In another embodiment of the invention, the facial mask has a Percent Increase in Transparency of at least about 50%. This improved facial mask allows the user to view the surface contact between the skin and the facial mask, providing better performance by the facial mask and improved aesthetics to the user.

While the article of the present invention will be described in the context of a facial mask, the mask may be readily adapted for use on other parts of the body, or used for other applications requiring a transparent substrate.

The phrase "mixtures" is meant to include a simple combination of materials and any compounds that may result from their combination.

All ingredients such as actives and other ingredients useful herein may be categorized or described by their cosmetic and/or therapeutic benefit or their postulated mode of action. However, it is to be understood that the actives and other ingredients useful herein can, in some instances, provide more than one cosmetic and/or therapeutic benefit or operate via more than one mode of action. Therefore, classifications herein are made for the sake of convenience and are not intended to limit an ingredient to the particularly stated application or applications listed.

### Water-Insoluble Substrate

The facial mask comprises a water-insoluble, nonwoven substrate. As used herein, "water-insoluble" means the substrate, upon immersion in distilled water at 25° C, does not readily dissolve or readily break apart. While portions of the substrate may be leachable or readily soluble in the distilled water, at least one portion of the water-insoluble substrate remains intact. For example, the intact portion may be readily manipulated, such as picked up and transported as an interconnected cohesive unit, by a user.

The water-insoluble substrate is formed to receive or preferably retain a wetting agent (such as by absorbing the wetting agent among, along, and/or between fibers comprising the water-insoluble substrate). Preferably, the water-insoluble substrate is capable of retaining the wetting agent from the time the product is manufactured to when the product is used by a consumer (i.e., a shelf storage period). In one embodiment of the invention, during this shelf storage period the water-insoluble substrate should generally maintain its mechanical integrity such that a user can lay the water-insoluble substrate onto the skin and transfer the wetting agent thereto. Furthermore, the water-insoluble substrate is generally capable of holding portions of the wetting agent against the facial skin when used.

**FIG. 1** depicts one embodiment of a facial mask consistent with embodiments of the invention described herein. Facial mask 1 is generally sized and shaped to lie against the face of a user. It is preferred that the facial mask 1 lie substantially flatly against the face of the user, i.e., the facial mask 1 is capable of draping across the face and generally conforming to the curvature of the face. The facial mask is generally further capable of lying substantially coincident with the face of the user, i.e., it generally only requires simple manipulation such as unfolding or at most slight tearing of optional, preformed perforations in order to assume a shape that coincides with a human face. In a preferred embodiment, the facial mask is sized and shaped to lie substantially flatly against the entire face of a user. As used herein, "entire face" means the majority, e.g., at least about 90%, of the face, including the nose, cheeks, around the eyes, and the forehead, as well as under and above the mouth. In another embodiment, the facial mask is sized and shaped to extend of over and lie substantially flatly against the chin and neck of a user.

The facial mask 1 may further comprise a vertical centerline, preferably a vertical axis of symmetry 2 that separates a left side 4 from a right side 6 of the facial mask 1. Furthermore, the facial mask 1 comprises a perimeter 3 that defines the outer boundary of the facial mask when it is laid flat such as on a tabletop.

The facial mask 1 may include, within the perimeter 3, at least one opening. The opening may be pre-cut, in which case it will be visible as area devoid of water-insoluble substrate. Alternatively, the opening may be defined by a perforated or weakened line. In this embodiment, the user can readily separate using little force and in a controlled fashion, the water-insoluble substrate along the perforated or weakened line. For example, a user may "punch" or "tear" the water-insoluble substrate along such a line or other shape in a controlled fashion prior to use.

As shown in **FIG. 1****,** the facial mask 1 includes pre-cut openings 7 for the eyes of a user. It is further desirable that facial mask 1 include a pre-cut opening 11 for the mouth of the user.

The facial mask may further include at least one interior slit. As used herein, an "interior slit" is a pre-cut line in the water-insoluble material within but not touching the perimeter. Interior slits are formed by slicing the water-insoluble substrate when laid flat and are often visible only as lines or boundaries when the facial mask is laid flat. For example, facial mask 1 includes one or more interior slits 13 that permit the nostril area or the mouth of the user to be exposed (for instance, uncovered) in use.

Interior slits 13 may be straight or curved. In one embodiment, in order to provide better fit, one or more interior slits 13 for the nose may include a plurality of arcuate portions to facilitate adherence to the underside of a user's nasal ridge, rather than, in the case of a user with a small nose, dangling or hanging off of the nose.

The facial mask 1 includes a forehead region 8 comprising that portion of the facial mask 1 entirely above the openings 7 for the eyes; a chin region 12 comprising that portion entirely below the opening 11 for the mouth; and a mid region 10 that includes all other portions of the facial mask 1.

Note that the perimeter 3 is generally of a gently curving, primarily arcuate shape that generally outlines or conforms to the shape of a typical human face. In accordance with the certain optional embodiments, the perimeter 3 may also encompass various features in order to enhance the fit and comfort for users having a wide range of facial shapes and sizes.

Specifically, in one embodiment, the facial mask may comprise of at least one separation feature. As used herein, a "separation feature" is a feature defined by a perforated or weakened line that can be readily separated by the user, or a pre-cut line, in the water-insoluble substrate that touches the perimeter. The separation feature may, for example, be a slit, notch or wedge, or other indentation or protrusion along the perimeter having a regular or irregular shape.

The water-insoluble substrate comprises a nonwoven material. As used herein, "non-woven" means that the substrate, or a layer of the substrate, is comprised of fibers that are not woven into a fabric but rather are formed into a sheet, mat, or pad layer. The fibers can either be random (i.e., randomly aligned) or they can be carded (i.e., combed to be oriented in primarily one direction). Furthermore, the water-insoluble substrate can be composed of a combination of layers of random and carded fibers.

Non-woven substrates may be comprised of a variety of natural and/or synthetic materials. By "natural" it is meant that the materials are derived from plants, animals, insects, or byproducts of plants, animals, and insects. By "synthetic" it is meant that the materials are obtained primarily from various man-made materials or from natural materials, which have been further altered. Non-limiting examples of natural materials useful in the present invention are silk fibers, keratin fibers (such as wool fibers, camel hair fibers) and cellulosic fibers (such as wood pulp fibers, cotton fibers, hemp fibers, jute fibers, and flax fibers).

Examples of synthetic materials include, but are not limited to, those selected from the group containing acetate fibers, acrylic fibers, cellulose ester fibers, cotton fibers, modacrylic fibers, polyamide fibers, polyester fibers, polyolefin fibers, polyvinyl alcohol fibers, rayon fibers, polyurethane foam, and mixtures thereof.

Water-insoluble substrates made from one or more of the natural and synthetic materials useful in the present invention can be obtained from a wide variety of commercial sources such as Freudenberg & Co. (Durham, NC USA), BBA Nonwovens (Nashville, TN USA), PGI Nonwovens (North Charleston, SC USA), Buckeye Technologies/Walkisoft (Memphis, TN USA), Sansho Shigyo K.K. (Tosa City, Kouchi, Japan), and Fort James Corporation (Deerfield, IL USA).

Methods of making water-insoluble substrates are also well known in the art. Such methods include, but are not limited to, air-laying, water-laying, melt-blowing, spin-bonding, or carding processes. The resulting substrate, regardless of its method of production or composition, is then generally subjected to at least one of several types of bonding operations to anchor the individual fibers together to form a self-sustaining web. The water-insoluble substrate can be prepared by a variety of processes including hydro-entanglement, thermal bonding, chemical bonding and combinations of these processes. Moreover, the water-insoluble substrate can have a single layer or multiple layers. In addition, a multi-layered water-insoluble substrate can include film layer(s) (e.g., aperture or non-aperture film layers) and other non-fibrous materials.

Strength or firmness of the non-woven material may be a desirable attribute. This can be achieved, for example, by the addition of binding materials, such as wet strength resins, or the material may be made of polymer binder coatings, stable fibers, e.g. based on cotton, wool, linen and the like. Examples of wet strength resins include, but are not limited to, vinyl acetate-ethylene (VAE) and ethylene-vinyl chloride (EVCL) Airflex emulsions (Air Products, Lehigh, PA), Flexbond acrylic polymers (Air Products, Lehigh, PA), Rhoplex ST-954 acrylic binder (Rohm and Haas, Philadelphia, PA), and Ethylene-vinyl acetate (EVA) emulsion (DUR-O-SET® by National Starch Chemicals, Bridgewater, NJ). The amount of binding material in the water-insoluble substrate may range from about 5% to about 20%, by weight, of the water-substrate substrate. In one embodiment, the water-insoluble substrate is flushable, i.e., the water-insoluble substrate will pass through at least 10 feet of waste pipe in two toilet flushes. The material may also be biodegradable.

Non-woven materials of increased strength can also be obtained by using the so-called spunlace or hydro-entanglement technique. In this technique, the individual fibers are twisted together so that an acceptable strength or firmness is obtained without the need to use binding materials. The advantage of the latter technique is the excellent softness of the non-woven material. In another embodiment, the water-insoluble substrate may include a hydrogel. By "hydrogel" it is meant a continuous network of polymer chains that are water-insoluble, sometimes found as a colloidal gel in which water is the dispersion medium, e.g., a multicomponent system consisting of a three-dimensional network of polymer chains and water that fills the space between macromolecules.

In one embodiment, the water-insoluble substrate is composed predominantly of a nonwoven fabric, a hydrogel, or combinations thereof; e.g., the water-insoluble substrate may be at least about 25% by weight (exclusive of any wetting agent) of such materials, more preferably at least about 50% by weight.

In another embodiment, water-insoluble substrate comprises from about 20% to about 90% by weight pulp, based on the total weight of the water-insoluble substrate. While the fibers may be staple fibers or continuous filaments, it is preferred that the fibers are staple fibers. It has been found that such levels of pulp impart the desired transparence to the water-insoluble substrate in accordance with the invention.

A particularly preferred pulp-containing material comprises a blend of 27% spun-bonded polypropylene, 44% pulp and 29% PET, 45 grams per square meter. It is commercially available from Orlandi SPA, Gallarate, Italy.

Furthermore, the water-insoluble substrate may include thermoplastic films (e.g., polyolefin) with or without apertures. In one embodiment, the water-insoluble substrate includes a stretchable or elastic material or film that is capable of fully recovering after being placed under tension of 50% or 100% strain, such as may be included for use on the laterally-extending tabs or across the entire facial mask.

In one embodiment the water-insoluble material has a basis weight that may range from about 10 grams per square meter (gsm) to about 200 gsm, such as between about 30 gsm and about 120 gsm, more preferably between about 45 gsm and about 90 gsm. The water-insoluble substrate may have an average thickness that is less than about 1.1 mm, such as between about 0.2 mm and about 2.0 mm.

In one embodiment of the invention, the non-woven material may include a superabsorbent polymer. For the purposes of the present invention, the term "superabsorbent polymer" refers to materials which are capable of absorbing and retaining at least about 10 times their weight in water under a 0.5 psi pressure. The superabsorbent polymer particles of the invention may be inorganic or organic crosslinked hydrophilic polymers, such as polyvinyl alcohols, polyethylene oxides, crosslinked starches, guar gum, xanthan gum, and other material known to the art of absorbent article manufacture.

Additives may also be added in order to increase the softness of the water-insoluble substrate. Examples of such additives include, but are not limited to, polyols such as glycerol, propylene glycol and polyethylene glycol, phthalate derivatives, citric esters, surfactants such as polyoxyethylene (20) sorbitan esters, and acetylated monoglycerides.

Sensory attributes may also be incorporated into the water-insoluble substrate. Examples of such sensory attributes include, but are not limited to color, texture, pattern, and embossing of the water-insoluble substrate.

The water-insoluble substrate when laid flat may cover an area that is from about 100 cm² to about 1000 cm², such as from about 200 cm² to about 500 cm², such as between about 200 cm² to about 360 cm².

### Wetting Agents

Exposure of the water-insoluble substrate to a wetting agent provides transparency to the facial mask. In one embodiment, the water-insoluble substrate and the wetting agent are provided separately and combined by the user just prior to or at the time of use. In another embodiment, the water-insoluble substrate and the wetting agent are provided to the user already combined, for example for a period of time such as the shelf storage period or a portion thereof.

In one embodiment, the wetting agent is present in an amount sufficient to saturate the water-insoluble substrate and wet a user's facial skin when laid onto such skin. In another embodiment, the wetting agent is present in an amount sufficient to permit a first portion of the water-insoluble substrate to cohesively attach to a second portion of the water-insoluble substrate, wherein the first portion of the water-insoluble substrate is distant from the second portion of the water-insoluble substrate. Such cohesive attachment may be for a period of time of at least about 5 minutes when the water-insoluble substrate is laid on the face and the user face is in an upright, vertical orientation.

In order to provide sufficient transparency of the water-insoluble substrate, the wetting agent may be used in an amount that is at least about 50% by weight of the weight of the water-insoluble substrate alone. More preferably the wetting agent is present in an amount that is at least about 75%, even more preferably at least about 85%, even more preferably at least about 200%, such as about 50% to about 5000%, for example about 200% to about 5000%, or from 50 to 300%, by weight of the weight of the water-insoluble substrate alone. This amount of wetting agent may be readily transferred to skin placed in contact with the water-insoluble substrate, as well as provide sufficient transparency to the facial mask. To further enhance the transfer of the wetting agent to the skin of the user, such as for a hydrating facial mask, the wetting agent may be present in an amount greater than about 50% by weight, such as greater than about 65%, such as between about 65% to about 95% by weight of the water-insoluble substrate alone.

The wetting agent may include an aqueous phase, an oily/hydrophobic phase, a gel phase, or a mixture of these phases. In one desirable embodiment, the wetting agent includes an aqueous phase, and even more preferably, the aqueous phase is an external phase (in which an oily phase or particulate phase may be dispersed, suspended or emulsified). In another desirable embodiment, the wetting agent includes an oil-in-water micro-emulsion for example comprising a polyacrylate co-polymer.

In one embodiment, the wetting agent has a viscosity that is less than about 5000 centipoise (cps), when measured using a Brookfield digital viscometer, Model DV-II+ Version 3.2 according to the operating instructions set forth in Manual No. M/92-161-H895, such as having a viscosity less than about 2500 cps, such as less than about 500 cps. Such low viscosity wetting agents tend to be aesthetically pleasing to the user.

The wetting agent may include solvents such as water, including those that are humectants such as glycols including glycerin or propylene glycol, or alcohols such as isopropyl alcohol or ethanol. In one preferred embodiment, the wetting agent includes water, such as in a concentration of at least about 40%, more preferably at least about 60%, and even more preferably at least about 90% by weight of the wetting agent.

The wetting agent may include any of various ingredients known in the art of facial mask preparations, including: hydrophobic emollients such as fatty esters including esters of glycerin, fatty alcohols, hydrophobic polymeric emollients; sensory agents such as menthol and methyl lactate, chelating agents such as EDTA, preservatives such as parabens, and other conventional cosmetic adjuvants, such as dyes, opacifiers (e.g., titanium dioxide and zinc oxide), pigments, fragrances, and microcapsules, such as aminoplast microcapsules. One particular example of suitable microcapsules are polyoxymethyene melamine urea (PMU) microcapsules, commercially available as Pontenza Dimethicone from Reed-Pacific of Dural, Austrialia. Another such example is PMU Microcapsules (32 Micron Encapsulated Mineral Oil and Jojoba Oil), available from 3M Company of St. Paul, MN.

### Transparency

According to one embodiment of the invention, at least a portion of the water-insoluble substrate has an Average Wet Transparency Percentage of at least about 25%, such as at least about 30% or preferably at least about 40%. Average Wet Transparency Percentage indicates the level of transparency of the substrate when exposed to a wetting agent.

In another embodiment of the invention, at least a portion of the water-insoluble substrate has Percent Increase in Transparency of at least about 50%, such as at least about 60% or preferably at least about 70%. Percent Increase in Transparency indicates the degree of change in transparency of the substrate between a dry condition and a wet condition.

Both the Average Wet Transparency Percentage and Percent Increase in Transparency indicate the ability of the substrate to transmit light, so that objects or images underneath can be seen as if there were less or no material present.

Average Wet Transparency Percentage and Percent Increase in Transparency may be measured using a SwissTester Transparency Measuring Device. The device has the following parts: a light sensor, a light source, a digital output and a calibration control means. The light sensor and the light source are magnetic and can be coupled to form a tight seal. The device is calibrated prior to use to read 100% transparency. The light sensor portion of the device is placed on a flat surface. A sample of material is then placed over the light sensor. The light source portion of the device is then placed on top of the sample and the transparency percentage value is read. Four readings are taken for each sample, each time repositioning a different area of the sample over the sensor. The average value is then calculated.

Average Wet Transparency Percentage is calculated by performing the above process on a sample of material saturated with a desired wetting agent. The average of the four values is the Average Wet Transparency Percentage of the material.

Percent Increase in Transparency is obtained by performing the above process on a sample in both dry and wet conditions, and subtracting the dry average transparency value from the wet average transparency value (Average Wet Transparency Percentage) to compare the difference.

### Benefit Agents

In one embodiment of the invention, the water-insoluble substrate or wetting agent includes one or more benefit agents. What is meant by a "benefit agent" is a compound (e.g., a synthetic compound or a compound isolated from a natural source) that has a cosmetic or therapeutic effect on the skin including, but not limited to, lightening agents, darkening agents such as self-tanning agents, anti-acne agents, shine control agents, anti-microbial agents, antiinflammatory agents, antifungals, anti-parasite agents, external analgesics, sunscreens, photoprotectors, antioxidants, keratolytic agents, moisturizers, nutrients, vitamins, energy enhancers, anti-perspiration agents, astringents, deodorants, hair growth inhibitors, anti hair-loss agents, hair growth promoters, hair removers, skin-firming agents, anti-callous agents, anti-aging agents such as anti-wrinkle agents, skin conditioning agents, allergy inhibitors, antiseptics, external analgesics, antipruritics, antihistamines, antiinfectives, anticholinergics, vasoconstrictors, vasodilators, wound-healing promoters, peptides, polypeptides, proteins, deodorants, anti-perspirants, film-forming polymers, counterirritants, enzymes, enzyme inhibitors, poison ivy treatment agents, poison oak treatment agent, burn treatment agents; anti-diaper rash treatment agents; prickly heat agents; herbal extracts; flavenoids; sensates; antioxidants, keratolytics; sunscreens; and anti-edema agents; and combinations thereof.

In one embodiment of the invention, the benefit agent is selected from, but not limited to, hydroxy acids, benzoyl peroxide, sulfur resorcinol, ascorbic acid and its derivatives, D-panthenol, hydroquinone, octyl methoxycinnimate, titanium dioxide, octyl salicylate, homosalate, avobenzone, polyphenolics, carotenoids, free radical scavengers, spin traps, retinoids such as retinol and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper, coenzyme Q 10, lipoic acid, amino acids such a proline and tyrosine, lipo amino acids such as capryloyl glycine and sarcosine, vitamins, lactobionic acid, acetyl-coenzyme A, niacin, riboflavin, thiamin, ribose, electron transporters such as NADH and FADH2, and other botanical extracts, and salt, esters, and derivatives thereof. The benefit agent will typically be present in an amount of from about 0.001% to about 20% by weight of the liquid impregnate, e.g., about 0.0 1 % to about 10% such as about 0.1 % to about 5%.

Examples of vitamins include, but are not limited to, vitamin A, a vitamin B such as vitamin B3, vitamin B5, and vitamin B 12, vitamin C, vitamin K, and vitamin E, and salts, esters, and derivatives thereof. (e.g., retinyl palmitate, ascorbyl acetate, and tocopherol acetate). Examples of hydroxy acids include, but are not limited, to glycolic acid, lactic acid, malic acid, salicylic acid, citric acid, and tartaric acid.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbic acid glucoside, magnesium ascorbyl phosphate, and ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isofavonoids and their derivatives (e.g., genistein and diadzein), extracts containing resveratrol and the like. Examples of such natural extracts include grape seed, green tea, pine bark, and propolis.

Examples of botanical extracts include, but are not limited to legumes such as Soy, Aloe Vera, Feverfew, Hedychium, Rhubarb, Portulaca, Cedar Tree, Cinnamon, Witch Hazel, Dandelion, Chinese Angelica, Turmeric, Ginger, Burnet, Houttuynia, Coix Seed, and Thyme. What is meant by a "botanical extract" is a blend of two or more compounds isolated from a plant.

In one embodiment of the invention, the benefit agent is designed for application on the forehead region and includes, but is not limited to: oil-control agents such as titanium dioxides, alcohols, botanical extracts, and talc; pore refining agents such as alpha-hydroxy acids, beta-hydroxy acids, and enzymes; anti-acne agents such as benzoyl peroxide, salicylic acid, trichlorcarban, triclosan, azelaic acid, clindamycin, adapalene, erythromycin, sodium sulfacetamide, retinoic acid, and sulfur; oil-absorbing agents such as titanium dioxides and clays; shine control agents such as silicones, alcohols, talc, and clays; dark spot reduction agents such as vitamin C, hydroquinone, botanical extracts, alpha-hydroxy acids, beta-hydroxy acids, and retinoids; and/or wrinkle/fine-line reduction agents such as retinoids, alpha-hydroxy acids, and enzymes.

In another embodiment of the invention, the benefit agent is designed for application around the mouth and includes, but is not limited to: hydration/moisturization agents such a glycerin, silicone, glycols, botanical extracts, and esters; pore-refining agents; anti-acne agents; vasodilators such as niacinamide and horsechesnut extract; vasoconstrictors such as caffeine and botanical extracts; skin-lifting agents such as (e.g., copper containing peptides, dimethyaminoethanol, and polymers); skin-firming polymers; wrinkle/fine-line reduction agents; depigmenting/skin lightening agents such as vitamin C, hydroquinone, botanical extracts, alpha-hydroxy acids, beta-hydroxy acids, retinoids, arbutin, and kojic acid; and depilatory/hair reducing agents such as soy extracts, n-acetyl-cysteine, and isoflavones.

While various combinations are contemplated, under one non-limiting example, one or more benefit agents are selected from the group consisting of ascorbic acid and its derivatives, alpha-hydroxy-acids, beta-hydroxyacids, alkanolamines, proteins, enzymes, and enzyme activators, and combinations thereof are in the liquid impregnate, and one or more benefit agents are selected from the group consisting of retinoids, tocopherols, enzymes, enzyme activators, and combinations thereof are within the liquid core.

In one embodiment of the invention, the product comprises an enzyme such as a lignin peroxidase and a suitable activator such as a peroxide (e.g., hydrogen peroxide) as described in WO 2004/052275.

### Packaging of Product

In one embodiment of the invention, the facial mask is a product is in finished packaged form inside a package. In one embodiment, the package is a container such as a plastic, metal or glass tube, tub, pouch or jar containing the water-insoluble substrate. The product may further contain additional packaging such as a plastic or cardboard box for storing one or more of such containers (e.g., a package of two to twenty individual products). Non-limiting examples of material that may be used to manufacture such containers include aluminum, polypropylene, polyethylene, and/or polyesters. In one embodiment of the invention, the package is substantially air-impermeable.

In one embodiment, where the water-insoluble substrate contains a wetting agent that is present in an amount of at least about 75% by weight of the weight of the water-insoluble substrate, the instructions direct the user to apply the product directly to the skin. In another embodiment where the water-insoluble substrate contains a wetting agent that is present in an amount less than about 75% by weight of the weight of the water-insoluble substrate or the product does not contain any wetting agent, the instructions direct the user to apply wetting agent to the facial mask prior to application to the skin.

In one embodiment, the instructions direct the user to apply the product for the benefit of changing the appearance of the tone and/or color of the skin.

The instructions may direct contacting the facial mask with the skin (e.g., the face) for a period of time, such as from about 10 seconds to about 1 hour (e.g., such as from about 1 minute to about 15 minutes). The user may also be directed to massage into the skin any liquid remaining on the skin after removal of the water-insoluble substrate. Such massaging may facilitate imparting improved color/tone uniformity in the skin of the subject.

### Method of Making and Using the Product

Facial masks of the present invention may be made by various conventional methods, known to those skilled in the art. For example, a water-insoluble substrate, such as a sheet of non-woven material optionally perforated or cut to a pre-determined size to form a "blank," such a size and shape suitable to fit over a human face, may be formed by methods well known in the art. Openings may optionally be cut out of the blanks corresponding to the eyes, nose, and/or mouth. Using a "subtractive" method one or more separation features such as slits, notches, and wedges may be sliced, cut or punched out of the blank. In one embodiment, the separation feature is formed by weakening or perforating the water-insoluble substrate rather than by slitting or removing material. Consistent with these embodiments, the facial mask may be of an "integrated" structure, in that the entire facial mask is essentially uniform throughout its area, as viewed from the top in a plan view.

In an alternative embodiment, the facial mask may be at least partially formed by an "additive" process, i.e., portions of the mask are stitched, bonded, or adhered together to create the separation features. After forming the water-insoluble substrate, the substrate may then be folded and placed in a plastic pouch housing or other suitable container.

It is generally preferred that facial masks of the present invention are unitary, i.e., a one-piece mask that can be readily picked up with a user's hands and transported as single cohesive unit, and this single cohesive unit is generally adapted to cover substantially the entire face of a user. However, the facial mask may also be formed from two or more pieces. Suitable two piece mask designs with slits include a top piece that includes most or all of the forehead portion and a bottom piece that includes most or all of the chin portion.

The wetting agent may be prepared by mixing ingredients such as water and one or other ingredients and/or more benefit agents together to form a uniform liquid. The resulting wetting agent may then be poured into the packaging. Alternatively, the agent may be sprayed onto or otherwise distributed in or on about the water-insoluble substrate.

The resulting facial mask may be individually sealed in the packaging or placed along with other water-insoluble substrates together into a single package. Multiple packaged facial masks may be grouped together in an outer container, such as a box.

The following examples illustrate details of embodiments of the invention, without limiting it in any matter.

### Example 1

A wetting agent of the present invention was prepared by combining the materials listed in Table 1 as follows. First, an oil phase was prepared by mixing and heating (40-45°C) phenoxyetol in a premix container. Then fragrance RP-2406, Dermosoft 688, Lamesoft PO 65, and Eumulgin VL 75 were added and mixed in until uniform. Second, in a separate mixture, a water phase was prepared by mixing water, Emery 917 and Hispagel 200 until the ingredients dissolved. Then Carbopol Ultrez 10 was added and mixed until uniform. The ingredients from the oil phase were then added to the water phase and mixed until uniform. Then 30% sodium hydroxide solution, Sodium Benzoate Dense NF/FCC and 20% Citric Acid solution were added and mixed until uniform.

**Table 1**

| **Ingredient** | **%W/W** |
|---|---|
| Phenoxyetol | 0.600 |
| Fragrance RP-2406 | 0.100 |
| Dermosoft 688 | 0.060 |
| Lamesoft PO 65 | 0.250 |
| Eumulgin VL 75 | 0.250 |
| Purified Water | 97.260 |
| Emery 917 | 0.180 |
| Hispagel 200 | 0.200 |
| Carbopol Ultrez 10 | 0.200 |
| 30% sodium hydroxide solution | 0.200 |
| Sodium Benzoate Dense NF/FCC | 0.500 |
| 20% Citric Acid | 0.200 |

### Example 2

A series of substrates were measured for Average Wet Transparency Percentage and Percent Increase in Transparency using the method described above as follows.

Each sample of substrate was cut to have outer dimensions of about 6.35 cm X 7.32 cm. Where a wetting agent was used, the substrate sample was placed in a weigh boat filled with 100 grams of wetting agent for thirty seconds to saturate. Each substrate was tested under four conditions: (1) in a dry state, (2) using water as a wetting agent, (3) using the liquid from a commercially available Neutrogena® Deep Hydrating Mask as the wetting agent, and (4) using the wetting agent of Table 1. The results are shown in Table2.

**Table 2**

| **SUBSTRATE** | **WETTING AGENT** | **Trial 1** | **Trial 2** | **Trial 3** | **Trial 4** | **AVERAGE WET TRANSPARENCY PERCENTAGE** | **PERCENT INCREASE IN TRANSPARENCY** |
|---|---|---|---|---|---|---|---|
| | | | | | | | |
| 45 GSM (27% Polypropylene, 44% Pulp, 29% PET) | None | 16.2 | 16.2 | 15.7 | 16.3 | 16.1 | |
| 45 GSM (27% Polypropylene, 44% Pulp, 29% PET) | Water | 25.7 | 25.0 | 26.6 | 24.7 | 25.5 | 58.4 |
| 45 GSM (27% Polypropylene, 44% Pulp, 29% PET) | Neutrogena® Deep Hydrating Mask liquid | 20.8 | 20.9 | 20.4 | 20.3 | 20.6 | 28.0 |
| 45 GSM (27% Polypropylene, 44% Pulp, 29% PET) | Table 1 Wetting Agent | 26.4 | 26.3 | 26.0 | 25.7 | 26.1 | 62.1 |
| | | | | | | | |
| 80 GSM (27% Polypropylene, 44% Pulp, 29% PET) | None | 8.9 | 9.0 | 8.9 | 9.0 | 9.0 | |
| 80 GSM (27% Polypropylene, 44% Pulp, 29% PET) | Water | 18.7 | 18.6 | 18.2 | 18.6 | 18.5 | 107.0 |
| 80 GSM (27% Polypropylene, 44% Pulp, 29% PET) | Neutrogena® Deep Hydrating Mask liquid | 16.3 | 16.5 | 16.7 | 16.9 | 16.6 | 85.5 |
| 80 GSM (27% Polypropylene, 44% Pulp, 29% PET) | Table 1 Wetting Agent | 17.0 | 16.3 | 16.2 | 16.4 | 16.5 | 84.1 |
| (55% Pulp, 35% Rayon, 10% PET) 75 GSM | None | 9.7 | 9.1 | 9.4 | 8.5 | 9.2 | |
| (55% Pulp, 35% Rayon, 10% PET) 75 GSM | Water | 17.2 | 17.6 | 17.5 | 17.2 | 17.4 | 89.4 |
| (55% Pulp, 35% Rayon, 10% PET) 75 GSM | Neutrogena® Deep Hydrating Mask liquid | 17.8 | 18.2 | 19.0 | 18.3 | 18.3 | 99.7 |
| (55% Pulp, 35% Rayon, 10% PET) 75 GSM | Table 1 Wetting Agent | 21.3 | 19.3 | 20.5 | 18.0 | 19.8 | 115.5 |
| | | | | | | | |
| Airlaid 95 GSM (80% Pulp) | None | 8.0 | 8.2 | 8.4 | 8.1 | 8.2 | |
| Airlaid 95 GSM (80% Pulp) | Water | 18.8 | 18.6 | 18.0 | 18.4 | 18.5 | 125.7 |
| Airlaid 95 GSM (80% Pulp) | Neutrogena® Deep Hydrating Mask liquid | 16.9 | 16.0 | 16.7 | 16.6 | 16.6 | 102.4 |
| Airlaid 95 GSM (80% Pulp) | Table 1 Wetting Agent | 17.5 | 18.5 | 17.2 | 17.4 | 17.7 | 115.9 |
| | | | | | | | |
| (60% Viscose, 40% PET) 50 GSM-microcreped | None | 13.8 | 14.0 | 14.0 | 14.5 | 14.1 | |
| (60% Viscose, 40% PET) 50 GSM-microcreped | Water | 18.4 | 19.7 | 18.7 | 18.4 | 18.8 | 33.6 |
| (60% Viscose, 40% PET) 50 GSM-microcreped | Neutrogena® Deep Hydrating Mask liquid | 19.1 | 17.9 | 19.2 | 18.1 | 18.6 | 32.0 |
| (60% Viscose, 40% PET) 50 GSM-microcreped | Table 1 Wetting Agent | 17.8 | 18.6 | 18.7 | 17.0 | 18.0 | 28.1 |
| | | | | | | | |
| Spunlace (40% Viscose, 60% PET) 50 GSM | None | 19.4 | 20.8 | 18.5 | 18.4 | 19.3 | |
| Spunlace (40% Viscose, 60% PET) 50 GSM | Water | 22.8 | 23.7 | 24.0 | 24.6 | 23.8 | 23.3 |
| Spunlace (40% Viscose, 60% PET) 50 GSM | Neutrogena® Deep Hydrating Mask liquid | 18.2 | 18.1 | 18.5 | 18.5 | 18.3 | -4.9 |
| Spunlace (40% Viscose, 60% PET) 50 GSM | Table 1 Wetting Agent | 20.6 | 22.5 | 21.5 | 22.3 | 21.7 | 12.7 |
| | | | | | | | |
| (40% PET, 60% Rayon) 45 GSM | None | 16.0 | 15.9 | 17.7 | 16.7 | 16.6 | |
| (40% PET, 60% Rayon) 45 GSM | Water | 22.0 | 20.7 | 19.5 | 21.1 | 20.8 | 25.6 |
| (40% PET, 60% Rayon) 45 GSM | Neutrogena® Deep Hydrating Mask liquid | 22.1 | 22.1 | 21.7 | 22.9 | 22.2 | 33.9 |
| (40% PET, 60% Rayon) 45 GSM | Table 1 Wetting Agent | 21.1 | 25.5 | 24.6 | 22.2 | 23.4 | 40.9 |
| | | | | | | | |
| (60% PET, 40% Rayon) 45 GSM | None | 19.9 | 20.3 | 21.2 | 20.5 | 20.4 | |
| (60% PET, 40% Rayon) 45 GSM | Water | 21.6 | 23.7 | 25.1 | 24.8 | 23.8 | 16.24 |
| (60% PET, 40% Rayon) 45 GSM | Neutrogena® Deep Hydrating Mask liquid | 23.8 | 24.4 | 25.3 | 23.1 | 24.1 | 17.95 |
| (60% PET, 40% Rayon) 45 GSM | Table 1 Wetting Agent | 23.9 | 24.6 | 24.4 | 26.5 | 24.8 | 21.37 |
| | | | | | | | |
| (60 PET, 40% RAYON) 52 GSM - microcreped | None | 13.2 | 11.8 | 13.5 | 13.2 | 12.9 | |
| (60 PET, 40% RAYON) 52 GSM - microcreped | Water | 18.7 | 19.2 | 19.2 | 18.9 | 19.0 | 47.0 |
| (60 PET, 40% RAYON) 52 GSM - microcreped | Neutrogena® Deep Hydrating Mask liquid | 19.6 | 19.0 | 19.5 | 19.7 | 19.5 | 50.5 |
| (60 PET, 40% RAYON) 52 GSM - microcreped | Table 1 Wetting Agent | 20.8 | 17.9 | 19.1 | 18.8 | 19.2 | 48.2 |
| | | | | | | | |
| (60% PET, 40%Rayon) 52 GSM-hydroembossed | None | 18.6 | 18.9 | 20.0 | 17.8 | 18.8 | |
| (60% PET, 40% Rayon) 52 GSM-hydroembossed | Water | 24.0 | 24.6 | 25.0 | 24.8 | 24.6 | 30.7 |
| (60% PET, 40% Rayon) 52 GSM-hydroembossed | Neutrogena® Deep Hydrating Mask liquid | 20.2 | 17.7 | 23.4 | 20.1 | 20.4 | 8.1 |
| (60% PET, 40% Rayon) 52 GSM-hydroembossed | Table 1 Wettir Agent | 23.9 | 22.6 | 22.0 | 21.4 | 22.5 | 19.4 |

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A facial mask for treating skin, comprising a water-insoluble, nonwoven substrate sized and shaped to lie against a face of a human user, wherein at least a portion of said water-insoluble substrate has an Average Wet Transparency Percentage of at least about 25%, and/or a Percent Increase in Transparency of at least about 50%.

2. The facial mask according to claim 1, wherein said water-insoluble substrate comprises a web selected from hydro-entangled nonwoven web, thermal bond nonwoven web, chemically-bonded dry-formed nonwoven web, or combinations thereof.

3. The facial mask according to claim 2, wherein said water-insoluble substrate comprises from about 20% to about 95% by weight of pulp based on the weight of said water-insoluble substrate.

4. The facial mask according to any preceding claim, wherein said wetting agent is an oil-in-water emulsion.

5. The facial mask according to claim 4, wherein said emulsion is combined with said water-insoluble substrate in an amount of about 50% to about 5000% or from about 50% to about 300% by weight based on the weight of said water-insoluble substrate alone.

6. The facial mask according to any preceding claim, wherein said water-insoluble substrate has a basis weight of about 10 gsm to about 200 gsm.

7. The facial mask of any preceding claim, wherein said water-insoluble substrate further comprises openings for the eyes and mouth of the user.

8. The facial mask of any preceding claim, wherein said water-insoluble substrate and said wetting agent are packaged together.

9. A cosmetic method of treating facial skin, comprising applying to said skin a facial mask according to any one of claims 1 to 7.
